(19) Europäisches Patentamt
European Patent Office
Office européen des brevets

(11) **EP 2 208 997 A2**

(12) **EUROPEAN PATENT APPLICATION**

(43) Date of publication:
21.07.2010 Bulletin 2010/29

(51) Int Cl.:
**G01N 33/50** (2006.01)　　**C07K 14/47** (2006.01)

(21) Application number: **09165838.5**

(22) Date of filing: **09.11.2004**

(84) Designated Contracting States:
**AT BE BG CH CY CZ DE DK EE ES FI FR GB GR HU IE IS IT LI LU MC NL PL PT RO SE SI SK TR**

(30) Priority: **13.11.2003 US 712629**

(62) Document number(s) of the earlier application(s) in accordance with Art. 76 EPC:
**04810571.2 / 1 685 402**

(27) Previously filed application:
**09.11.2004 PCT/US2004/037263**

(71) Applicant: **The Procter and Gamble Company Cincinnati, Ohio 45202 (US)**

(72) Inventors:
- **Sreekrishna, Kotikanyadanam Mason, OH 45040 (US)**
- **Leboeuf, Robert, Alexander Cincinnati, OH 45242 (US)**

(74) Representative: **Hampton, Matthew John et al Howrey LLP Rembrandt Tower, 31st Floor Amstelplein 1 1096 HA Amsterdam (NL)**

Remarks:
This application was filed on 17-07-2009 as a divisional application to the application mentioned under INID code 62.

(54) **Hairless protein-interacting partner complexes and methods thereof for the beautification and/or improvement of mammalian skin**

(57) Hairless protein interacting partner complexes, agonist and / or antagonist compounds, compositions and products comprising same and methods thereof. Hairless protein is indispensible for the beautification and / or improvement of mammalian skin. Therefore, such interacting complexes are useful in screening test compounds for the beautification and / or improvement of mammalian skin.

EP 2 208 997 A2

**Description**

Field of Invention

The present invention relates to hairless protein-interacting partner complexes, antagonist or agonist active compounds, compositions and products comprising same and methods thereof. The complexes are useful for screening test agents and identifying compounds that convey material beautification and improvement benefits to mammalian, and particularly human, skin.

Background of the Invention

Humans continue to demonstrate an obsession with appearance, particularly of the face, but increasingly of the skin and body as well. Indeed, many believe that appearance is intrinsically linked to self-esteem, the selection of a significant other, professional advancement and overall societal acceptance. The condition of human skin typically peaks at the age of 20, with subsequent thinning and loss of elasticity. Not surprisingly, American consumers spent approximately $2.8 billion in 2001 on products that claimed to slow or reverse the aging of skin. The demand for appearance-enhancing modalities continues to increase, as evidenced by the advent of many new products and services, each of which purports to achieve a desired appearance-enhancing result.

Lotions (herbal and otherwise), claiming a variety of skin benefits, are introduced to the skin care market globally every year. Other products having well-defined chemical entities (*e.g.*, alpha-hydroxy acids, niacinamide, retinoids, vitamin E, kinetin, Copper-peptide) are also widely available. Despite the commercialization of such products, however, there remains a significant and unmet need for compositions and products that are useful in the prevention and reversal of skin aging. Indeed, various clinical and/or surgical procedures, including injections (*e.g.*, botulin toxin, collagen, hyaluronic acid, keratin, silicone, cadaver skin extract) have proven to be effective in the beautification of mammalian skin. Nevertheless, such procedures tend to be expensive and time consuming, and thus, generally unavailable to most consumers. Further, as with other medical procedures, clinical and surgical approaches to the beautification of mammalian skin are associated with many risks and side effects. Herbal remedies, also found in the global skin care market, have not yet been demonstrated to convey material beautification benefits to mammalian skin. Moreover, recent adverse findings regarding hormone replacement therapy have limited the viability of drug-based therapies in the beautification of mammalian skin. Although neutraceuticals (or functional foods) may prove effective in the context of skin beautification, considerable progress is required for the identification of safe and efficacious compounds with skin anti-aging benefits.

Thus, despite the immense efforts exerted by those skilled in the art, there remains a substantial and unmet need in the identification of age-defying and age-reversing products, and particularly compounds that are both efficacious and safe. For instance, estrogen provides unique skin benefits, but has numerous undesirable side effects due to its diversified role in other tissues - thereby limiting its use in a cosmetic skin care context. Estrogen effects are generally mediated by numerous estrogen receptors (*e.g.*, ER$\alpha$, ER$\beta$, ERx) and estrogen receptors themselves are known to interact with other cellular proteins in a variety of tissues. This dilemma underscores the need to identify skin benefit agents that are specific to a cell or tissue type of interest. Contemporary advancements in genomic-proteomic and bioinformatic tools now facilitate the discovery of cell and/or tissue-type events via classical molecular genetic and biochemical approaches. Protein complexes generally control biological processes, and thus, the identification of skin-specific protein complexes and/or interaction partners encourages the discovery of skin anti-aging 'targets' and skin compounds with skin anti-aging benefits.

Hairless protein is a putative GATA family Zinc finger protein, for which several interaction partners exist. Indeed, the diversified interaction partners of HR continue to present a challenge and opportunity for pathway mapping and the discovery of certain skin and hair-enhancing products. Those of skill in the art have, to date, only identified a relationship between hairless protein and mammalian hair growth and maintenance. It has been recognized that individuals with point mutations in HR are hairless but are otherwise in good health. US Patent Number 6,348,348, issued February 19, 2002 to Thompson et al., discloses that HR interacts directly and specifically with thyroid hormone receptor (TR)-the same protein that regulates its expression. 6,348,348 further discloses a comparison of the amino acid sequences of the subject human HR sequence, a human sequence published by Ahmad et al. (Science, 279, 720-724, 1998); a human sequence published by Cichon et al. (Hum. Mol. Genet., 7, 1671-1679 and erratum at 1987-1988, 1998); a rat sequence published by Thompson (J. Neurosci., 16, 7832-7840, 1996); and a mouse sequence published by Cachon-Gonzalez et al. (Proc. Natl. Acad. Sci. USA, 91, 7717-7721, 1994). WO 99/38965 provides an isolated nucleic acid that encodes human hairless protein. Finally, commonly assigned US Patent Application serial number 10/452,858, filed on 02 June 2003 and entitled "Hairless Protein-Interacting Partner Complexes and Methods Thereof" describes the use of hairless protein-interacting partner complexes for the inhibition or enhancement of hair growth. Although those of skill in the art have identified some interaction between HR and Thyroid Hormone receptors (TRs), never hereinbefore has an interaction between HR and other proteins been identified in Keratinocytes of mammalian skin. Accordingly, there remains a substantial and unmet need in the art to develop specific protein complexes that can be used to screen test agents, and thus, identify compounds that provide one or more beautification benefits for mammalian skin.

Summary of the Invention

The present invention relates to hairless protein interacting partner complexes (HR-IP) for use in the screening and/or discovery of compounds adapted to provide material beautification and improvement benefits to mammalian skin. The present invention further relates to compounds exhibiting antagonist and/or agonist activity, compositions and products comprising said compounds and methods of using both the compounds and compositions disclosed herein to convey material beautification and/or improvement benefits to mammalian skin. As will become apparent from the present disclosure, the present inventors employed a modified, yeast two-hybrid technology to identify the hairless protein interacting partner complexes disclosed herein, the interaction of which is believed to have many implications upon the condition of mammalian skin. The hairless protein interacting partners provided by the present invention are listed in Table 1 and include molecules involved in cell cycle, cell differentiation, transcription, nuclear transport, signal transduction, cellular integrity, and mitochondrial machinery.

Thus, in one aspect of the present invention, a composition comprising a mouse HRt protein-human interacting partner protein complex wherein the human interacting partner protein comprises a molecule selected from the group consisting of Homo Sapiens Keratin 5; Oxysterols Receptor LXR-Beta; Homo Sapiens Protein Inhibitor of Activated STAT-1 (PIAS1); Homo Sapiens Similar to Stromal Antigen 2; Homo Sapiens Nucleoporin 160kD (NUP160); Human Retinoic Acid Receptor Gamma 1; Homo Sapiens Thyroid Hormone Receptor Alpha; Homo Sapiens Annexin A1 (Lipocortin-1 or P35); Homo Sapiens HIC Protein Isoform p32 and HIC Protein Isoform 40; Homo Sapiens Insulin-like Growth Factor Binding Protein 6; Homo Sapiens Inner Membrane Protein Mitochondrial (Mitofilin); Homo Sapiens Hypothetical Protein; Homo Sapiens Protein Inhibitor of Activated STAT-3 (PIAS3); Homo Sapiens DEAD/H (Asp-Glu-Ala-Asp/His) Box Polypeptide 3; Homo Sapiens Dpy-30-like Protein; Mouse Vitamin D Receptor (VDR) and combinations thereof, is provided. To reiterate, the aforementioned molecules are useful for screening test agents and identifying compounds that convey material beautification and improvement benefits to mammalian skin.

In another aspect of the present invention, a composition comprising a human interacting partner proteins encoded by a nucleic acid comprising SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15; SEQ ID NO 16; and combinations thereof, is disclosed.

In yet another aspect of the present invention, a method of assaying a test compound for agonist or antagonist activity in the beautification and/or improvement of mammalian skin is disclosed. Said method comprises the steps of a) measuring a level of interaction between mouse HRt protein and the human interacting partner in the absence of the test compound; b) measuring a level of interaction between mouse HR protein and the human interacting partner in the presence of the test compound; wherein when the level measured in step b) is greater than the level in step a), the test compound has agonist activity, and wherein when the level measured in step b) is less than the level in step a), the test compound has antagonist activity.

In another aspect of the present invention, compounds exhibiting agonist and antagonist activity, as hereinbefore defined, are disclosed and claimed. Said compounds may be identified in accordance with the present invention by employing the method of assaying a test compound, discussed *infra.* The agonist and antagonist compounds of the present invention may be delivered to a portion of mammalian skin for which the conveyance of material improvement and/or beautification benefits is desired. Said application and/or delivery may be achieved via a variety of ways, including but not limited to, the incorporation of said compounds into a topical composition and/or the use of a pharmaceutical carrier for oral administration.

In yet another aspect of the present invention, compositions incorporating one or more agonist and/or antagonist-active compounds of the present invention, are disclosed. Indeed, as will become apparent from reviewing the present disclosure, the test compounds of the present invention, whether exhibiting agonist or antagonist activity, may be formulated into a composition and/or incorporated into a product for the conveyance of material beautification and/or improvement benefits to mammalian skin. The compositions and/or products of the present invention may take a multitude of shapes, sizes and properties, depending on the precise needs of the formulator, the nature of the compound in question and the purpose for which employment of such a composition or product is intended. In another aspect of the present invention, the compositions and/or products disclosed herein may incorporate one or more adjunct ingredients, as discussed *infra*.

In yet another aspect of the present invention, a method of conveying material beautification and/or improvement benefits to mammalian, and particularly human skin, is disclosed. Said method comprises the step of administering, whether orally or topically, a compound demonstrating agonist and/or antagonist activity, as determined via employment of the previous method disclosed herein, for a time sufficient to convey material beautification and/or improvement benefits to mammalian skin. In another aspect of the present invention, said method involves the application of a composition or product comprising the agonist and/or antagonist compounds disclosed herein onto a portion of mammalian skin for which the conveyance of material beautification and/or improvement benefits is desired.

Detailed Description of the Invention

*Background and Definitions*

The present invention relates to hairless protein interaction partners (HR-IP), compounds exhibiting agonist and/or antagonist activity, compositions and products comprising same; and methods thereof. A modified, improved yeast two-hybrid system (discussed *supra*) was used to identify protein interaction partners with hairless protein. The yeast two-hybrid system, in general, measures the association of two fusion proteins expressed in yeast. Interaction discovered in yeast is indicative of potential interaction that would occur under physiological conditions.

By "hairless protein (HR)" is meant herein the mouse hairless protein. By "truncated hairless protein (HRt)" is meant the sequence provided as SEQ ID NO 16, which is amino acid residues 490-1182 of the C-terminal portion of mouse HR protein. Derivatives, fragments, or analogs of HR known to one of skill in the art in light of the present disclosure are considered equivalents of HR. It should be noted and underscored that mouse HR is greater than 80% identical to human HR. Thus, the interacting partners provided herein are expected to interact with human hairless protein in the same manner as such interacting partners interact within mouse HR. Accordingly, an antagonist and/or agonist compound having activity for HRt is expected to further exhibit activity for HR. Antagonists or agonists of the present HRt-IP complexes are further expected to exhibit activity for human hairless protein interacting partner equivalents.

By "interacting partner (IP)," is meant a protein or nucleic acid that has sufficient binding affinity for HRt such that, when the IP and HRt are fused into separate constituents of a transcriptional regulator, the HRt-IP affinity is sufficient to allow reconstitution of the constituents of the transcriptional regulator - thereby allowing expression of reporter gene. The interaction may occur due to specific electrostatic, hydrophobic, hydrophilic, entropic or other interaction of certain portions of HRt with certain portions of IP to form a stable complex under conditions effective to promote the interaction. Interacting partners are identified by the data provided in Table 1. That is, the identity of the interacting partner was determined by homology searching using nucleotide sequence data from positive results in the yeast two-hybrid system. Derivatives, fragments, or analogs of interacting partners identified in Table 1 known to one of skill in the art in light of the present disclosure are considered equivalents of interacting partners, and thus, are well within the scope of the present disclosure.

By "HRt-IP complex," it is meant that at least one molecule of HRt associated with at least one molecule of interacting partner, under physiological conditions of ionic strength, temperature, pH and the like. The complex may be *in vivo* or *in vitro.*

In the yeast two-hybrid system, transcription of a reporter gene (*e.g.*, LACZ, MEL1, HIS3, ADE1, URA3) is dependent upon reconstitution of a Gal 4 regulator by the interaction of two proteins, each fused to "half' of the regulator. When the two proteins have sufficient binding affinity to interact, the interaction results in the reconstitution of the GAL 4 regulator, which then activates transcription of the reporter gene(s). In the context of the present invention, "half" of the regulator is a DNA binding domain (BD), and "half" of the regulator is an activation domain (AD). When a "bait" protein is fused to the binding domain, and a "prey" protein is fused to the activator domain, or vice versa, sufficient binding affinity of the "bait" and "prey" proteins allow reconstitution of BD and AD to allow the reporter gene to be activated. Such an assay therefore is a test for affinity between the "bait" and "prey" proteins. The yeast two hybrid technology has been described in, for example, US Patent Number 5,986,055, issued November 16, 1999, to Yang, M., et al., incorporated herein by reference. The inventors of the present subject matter have employed a modified, yeast two hybrid technology, described in detail *infra.*

In the context of the present invention, the "bait" protein is a C-terminal portion of hairless protein of mouse (HRt) having amino acid residues 490 to 1182 (provided as SEQ ID NO:16, the nucleic acid sequence encoding amino acids 490 to 1182 is provided as SEQ ID NO:17) "Structure and Expression of the Hairless Gene of Mice," Begona, M., et al., J. Proc. Natl. Acad. Sci, USA 91:7717-7721, 1994) (GenBank accession no. Z32675). HR protein (aa residues 490 to 1182) contains the zinc finger domain (595-620) and three PEST domains previously identified to be potentially important for HR function. To reiterate, mouse HR very similar to human HR. Indeed, PEST sequences are often used to serve as signals for rapid protein degradation. The signals may be constitutive or conditional. The PEST-FIND computer program has been developed by M. Rechsteiner and S. W. Rogers (PEST sequences and regulation by proteolysis, TIBS 21, July 1996) to objectively determine whether a protein contains a PEST region. The associated algorithm, available in PC/GENE (OMIGA), defines a PEST sequence as a hydrophilic stretch of 12 or more aa residues containing at least one of each of P, E/D, and S/T in any order. PEST sequences must further be flanked by basic aa residues K, A or H, but basic residues are disallowed in the PEST sequence. PEST-FIND produces a score ranging from about +50 to -50. For classification purposes, a score of less than zero denotes a possible PEST region, but a score of greater than +5 denotes a high potential of the existence of a PEST region. For example, PEST Domains were located in the following regions of the HR protein (aa residues 490 to 1182): PEST Domain 12.26 between aa residues 522 and 546; PEST Domain 7.56 between aa residues 709 and 722; and PEST Domain 21.71 between aa residues 729 and 744.

In the context of the present invention, the "prey" proteins are encoded by a human Keratinocyte AD-cDNA library. When two complimentary mating types of yeast, one containing the BD-HRt fusion protein, and the other containing the AD-human cDNA protein, are mated, the progeny express each fusion protein. Those AD-proteins having sufficient affinity for HRt will bind HRt and allow the BD and AD to reconstitute to activate a reporter gene. Therefore, positive results from the two-hybrid system demonstrate affinity and interaction between the mouse HRt and a human Keratinocyte protein.

By an "antagonist" of HR-IP interaction, is meant an agent having inhibitory activity for the binding of the HR-IP complex. The binding may be inhibited by an effect on the interaction between HR and IP, or by an individual effect on HR or IP that affects the collective interaction between HR and IP.

By an "agonist" of HR-IP interaction, is meant an agent having enhancing or stiinulatory activity for the binding of the HR-IP complex. The binding may be stimulated by an effect on the interaction between HR and IP or by an individual effect on HR or IP that affects the collective interaction between HR and IP. Identification of an antagonist or an agonist is made by allowing HR and IP to interact in the presence of a test agent. A decrease or increase in HR-IP interaction relative to the interaction in the absence of the test agent generally indicates that the test agent has an affect on the interacting pair as further described *infra.*

By "beautification and/or improvement benefits," it is meant that the hairless protein interacting partner complexes of the present invention may be used to screen and/or identify compounds having antagonist and agonist activity for binding of the HR-IP complex, that convey one or more of the following benefits to mammalian skin: Improved epidermal barrier function and hydration, cellulite reduction, enhanced barrier repair, prevention as well as reversal of skin wrinkling and hair growth retardation.

Since mouse HR is greater than 80% identical to human HR, the present inventors would expect with reasonable certainty that the human proteins that interact with mouse HRt provided by the present invention would also interact with human HRt. Similarly, test compounds that prove to be agonists or antagonists of a mouse HRt-human interacting partner interaction are expected by the present inventors to be agonists or antagonists of the human HRt-human interacting partner.

In HR-interacting partner protein compositions of the present invention, conservative amino acid substitutions (*e.g.* Glu/Asp, Val/Ile, Ser/Thr, Arg/Lys and Gln/Asn) would be considered equivalent since the chemical similarity of these pairs of amino acid residues would be expected to result in functional equivalency. Amino acid substitutions that conserve the biological function of the HR-interacting partner protein would conserve such properties as hydrophobicity, hydrophilicity, side-chain charge, or size. Functional equivalency is determined by the interaction of the equivalent pair as compared to the native pair. Included within the scope of the invention are complexes of HR-interacting protein fragments, derivatives, or analogs that are modified during or after translation, *e.g.*, by glycosylation, acetylation, phosphorylation, amidation, fatty acylation, sulfation, derivatization by known protecting/blocking groups, proteolytic cleavage, linkage to an antibody molecule or other cellular ligand, or the like.

In the HR-interacting partner nucleic acid compositions of the present invention, a molecule that is the complement of a nucleic acid molecule that hybridizes to the interacting partner nucleic acid under stringent hybridization conditions would be considered equivalent since such a molecule is expected to result in functional equivalency as an interacting partner nucleic acid. Functional equivalency is determined by the interaction of the equivalent pair as compared to the native pair. Stringency conditions for hybridization depend upon a number of factors such as the length of hybridizing molecules, salt concentration, temperature, and the presence or absence of denaturing agents, for example. High stringency conditions may include hybridization at about 42°C and about 50% formamide, 0.1 mg/mL sheared salmon sperm DNA, 1% SDS, 2X SSC, 10% Dextran sulfate, a first wash at about 65°C, about 2X SSC, and 1% SDS, followed by a second wash at about 65°C and about 0.1X SSC. Alternatively, high stringency conditions may include hybridization at about 42°C and about 50% formamide, 0.1 mg/mL sheared salmon sperm DNA, 0.5% SDS, 5X SSPE, 1X Denhardt's, followed by two washes at room temperature and 2X SSC, 0.1 % SDS, and two washes at between 55-60°C and 0.2X SSC, 0.1 % SDS.

HR-IP complexes may be used for the preparation of polyclonal or monoclonal antibodies, antibody fragments, humanized antibodies, single chain antibodies for affinity purification, detection and/or other functional studies. Methods for producing antibodies are well known in the art and can be applied to HR-IP complexes in light of the present disclosure. Antibodies having binding specificity for the HR portion or the IP portion of the HR-IP complex may be removed from an anti-HR-IP preparation by adsorption with HR or with the IP. Remaining antibodies will have specificity for the complex.

*Background on Mouse HR Mutations*

The hairless (HR) gene is expressed in a large number of tissues, primarily the skin, and a mutation in the HR gene is responsible for the typical cutaneous phenotype of hairless mice. Mutant HR mouse strains show immune defects involving especially T cells and macrophages, as well as an age-related immunodeficiency and an accelerated atrophy of the thymus. (Ref: The thymus of the hairless rhino-j (hr/rh-j) mice. Jose et al. J Anat 2001 Apr;198(Pt 4):399-406

**Hairless mice (hr/hr):** The original hairless mutation was the first to be characterized at the molecular level. Murine leukemia virus pmv43 insertion in the intron 6 of HR gene causes aberrant splicing and a correctly spliced HR is expressed at approximately 5% of the normal level. Thus, this type of mouse expresses a small amount of the correct HR protein. (Ref: Role of endogenous retroviruses as mutagens: the hairless mutation of mice. Stoye etal. Cell 1988, 54:383-391).

**Rhino Yurlovo mice (hr^rhY):** A 13 bp insertion at nucleotide position 3520 in exon 16 of HR gene. This results in frame shift and premature termination at 264 bp downstream from the site of insertion in exon 17, and 139 bp upstream from the endogenous stop codon in exon 19. (Ref: Molecular basis for the rhino Yurlovo (hr^rhY) phenotype: Severe skin abnormalities and female reproductive defects associated with an insertion in the hairless gene. Panteleyev et al., Exp. Dermatol. 1998, 7:281-288)

**Rhino mice (hr^rh-8j):** A 2 bp substitution at nucleotide positions 1910 and 1911 of exon 4, resulting in a glutamine to histidine amino acid substitution at position 511 (Q511H) and a lysine to a nonsense codon at the adjacent amino acid 512 (K512X). Rhino is a functional knock-out of the HR gene. (Ref: Molecular basis for the rhino(hr^rh-8j) phenotype: A nonsense mutation in the mouse hairless gene. Ahmad et al., Genomics 1998, 53:383-386)

**Rhino mice (hr^rhChr):** C to T transition resulting in the conversion of an arginine residue (CGA) at amino acid position 467 (R467X) to a premature termination codon (TGA). (Ref: Molecular basis of a novel rhino (hr^rhChr) phenotype: a nonsense mutation in the mouse hairless gene. Ahmad et al. Exp. Dermatol. 1998, 7:298-301).

**Rhino mice (hr^rh/hr^rh):** Expresses a truncated version of HR (C-terminal amino acids 711-1189 are absent) due to premature stop in exon 6. (Ref: The hairless gene of the mouse: relationship of phenotypic effects with expression profile and genotype. Gonzalez et al. Dev Dyn. 1999 Oct; 216(2):113-26).

**Mutation Spectrum in human HR causing congenital atrichias**

| Mutation | | Location | Phenotype | Dermal cysts | Origin | Reference |
|---|---|---|---|---|---|---|
| p.R33X | | Exon2 | APL | Yes | Mediterranean | Zlotogorski et al., (2002a) |
| c.1256delC | | Exon 3 | AUC | Yes | Palestenian | Ahmad et al(1999a) |
| p.Q478X | | Exon 4 | APL | Yes | Pakistani | Sprecher et al (1999b) |
| p.R613X | | Exon 6 | APL | Yes | Japanese | Ahmad et al (1999b) |
| p.R620Q | | Exon 6 | AUC | Yes | Irish | Ahmad et al (1998b) |
| p.C622G | | Exon 6 | APL | Yes | Polish | Aitaet al (2000) |
| c.2001del CCAG | | Exon 7 | APL | Yes | Mexican | Kruse et al (1999) |
| c.2147delC | | Exon 9 | APL | Yes | Palestenian | Zlotogorski et al (1998) |
| c.2776 +1G>A | | Exon 12 | AUC | No | Omani | Cichon et al (1998) |
| p.N970S | | Exon 14 | APL | Yes | Tyrolian | Kruse et al (1999) |
| p.D1012N | | Exon 15 | AUC | Yes | Arab Israeli | Klein et al(2002) |
| p.T1022A | | Exon 15 | AUC | Yes | Pakistani | Ahmad et al (1998a) |
| p.V1056M | | Exon 16 | APL | Yes | Palestenian | Zlotogorski et al(2002b) |
| p.V1136D | | Exon 18 | AUC | Yes | Pakistani | Cichon et al (1998) |
| c.del3434C | | Exon 18 | APL | Yes | Arab Israeli | Sprecher et al (1999a) |
| Location of the mutation refers to GeneBank accession numbers AF039196 (nucleotide sequence) and NP-_005135 (amino acid sequence) APL= Atrichia with papularlesions AUC=alopecia universalis congenita | | | | | | |

References: Zlotogorski et al., (2002a), Clinical and molecular diagnostic criteria of congenital atrichia with papular lesions, J Invest Dermatol. 2002, 118:887-890. Ahmad et al(1999a), Genomic organization of the humanhairless gene (HR) and identification of a mutation underlying congenital atrichia in the Arab Palestinian family, Genomics 56, 141-148. Sprecher et al (1999b), Atrichia with popular lesions resulting from a nonsense mutation within the human hairless gene, J. Invest. Dermatol 113, 687-690. Ahmad et al (1999b), A homozygous nonsense mutation in thezinc-finger domain of the human hairless gene underlines congenital atrichia, J Invest Dermatol 113, 281-283. Ahmad et al (1998b), A miss sense mutation in the zinc-finger domain of the human hairless gene underlines congenital atrichia in a family of Irish travelers, Am J Hum Genet 63, 984-991. Aitaet al (2000), A novel missense mutation (C622G) in the zinc-finger domain of the human hairless gene associated with congenital atrichia and popular lesions, Exp Dermatol 9, 157-162. Kruse et al (1999), Hairless mutations in two kindreds with autosomal recessive popular atrichia, J Invest Dermatol 113, 954-959. Zlotogorski et al(1998), Congenital atrichia in five Arab Palestinian families resulting from a deletion mutation in the human hairless gene, Hum Genet. 1998,103:400-404. Cichon et al (1998), Cloning, genomicorganization, alternative transcripts and mutational analysis of the gene responsible for autosomal recessive universal congenital alopecia, Hum Mol Genet 7, 1671-1679. Kruse et al (1999), Hairless mutations in two kindreds with autosomal recessive popular tarichia, J Invest Dermatol 113, 954-959. Klein et al(2002), A novel missense mutation affecting the human hairless thyroid receptor interaction domain 2 causes congenital atrichia, J Invest Dermatol 119, 920-922. Ahmad et al (1998a), Alopecia universalis associated with mutation in the human hairless gene, Science 279, 720-724. Zlotogorski et al(2002b), Evidence for pseudodominant inheritance of atrichia with papular lesions, J Invest Dermatol 2002,118: 881-886. Sprecher et al (1999a), Identification of a genetic defect in the hairless gene in atrichia with popular lesions: evidence for phenotypic heterogeneity among inheritedatrichia, Am J Hum Genet 64, 1323-1329, all of which are incorporated herein by reference.

*First Aspect: HRt Protein-Human Interacting Partner Protein Complexes*

In accordance with a first aspect of the present invention, a composition comprising a mouse HRt protein-human interacting partner protein complex wherein the human interacting partner protein comprises a molecule selected from the group consisting of Homo Sapiens Keratin 5; Oxysterols Receptor LXR-Beta; Homo Sapiens Protein Inhibitor of Activated STAT-1 (PIAS1); Homo Sapiens Similar to Stromal Antigen 2; Homo Sapiens Nucleoporin 160kD (NUP160); Human Retinoic Acid Receptor Gamma 1; Homo Sapiens Thyroid Hormone Receptor Alpha; Homo Sapiens Annexin A1 (Lipocortin-1 or P35); Homo Sapiens HIC Protein Isoform p32 and HIC Protein Isoform 40; Homo Sapiens Insulin-like Growth Factor Binding Protein 6; Homo Sapiens Inner Membrane Protein Mitochondrial (Mitofilin); Homo Sapiens Hypothetical Protein; Homo Sapiens Protein Inhibitor of Activated STAT-3 (PIAS3); Homo Sapiens DEAD/H (Asp-Glu-Ala-Asp/His) Box Polypeptide 3; Homo Sapiens Dpy-30-like Protein; Mouse Vitamin D Receptor (VDR) and combinations thereof, is provided. The molecules disclosed herein have been identified employing a modified yeast two hybrid technology, described *infra.* Indeed, the aforementioned molecules are useful for screening test agents and identifying compounds that convey material beautification and/or improvement benefits to mammalian skin.

In another aspect of the present invention, human interacting partner proteins encoded by a nucleic acid comprising SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15; SEQ ID NO: 16; and combinations thereof, are provided. Said nucleic acid sequences are additionally useful for screening test agents and identifying compounds that convey material beautification and/or improvement benefits to mammalian, and particularly human skin. Compounds may be tested for agonist and/or antagonist activity toward the HR-IP complexes, including the molecules and nucleic acid sequences disclosed herein, in accordance with subsequent sections of the present disclosure.

*Second Aspect: Antagonist and/or Agonist Compounds of HR-IP Complexes*

In accordance with a second aspect of the present invention, compounds exhibiting antagonist and/or agonist activity toward HR-IP complexes are disclosed and claimed. Of course, the human interacting partner proteins described in the preceding section may be employed to screen antagonist and/or, agonist compounds that are adapted to bind to HR-IP complexes. The interaction of said compounds with the HR-IP complex facilitates their employment in compositions and products, consumer and otherwise, for the conveyance of one or more material beautification and/or improvement benefits to mammalian skin. The antagonist and/or agonist compounds of the present invention may be identified using a novel assay (described infra) and are then purified using other methods disclosed herein.

*Screening for Antagonists or Agonists of HR-IP Complexes*

HR-IP complexes or nucleic acids encoding HR-IP complexes may be used to screen for compounds that bind to HR-IP complexes and thus, such compounds have use as agonists or antagonists of HR-IP complexes. The invention therefore provides assays to detect molecules that either enhance or inhibit interaction of HR and an interacting partner. Such molecules may be small molecules, peptides, modified peptides, aptamers, nucleic acids, or modified nucleic acids, for example. A molecule that modulates activity of HR-IP is identified by contacting a test molecule either with HR or with IP prior to contacting HR and IP together, or allowing a three way binding among HR, IP and the test molecule. Modulation of HR-IP interaction may be measured by measuring the stability of the complex, the affinity or binding of the complex, rate of formation of the complex, the amount of complex, or another function of the complex. An increase or decrease in any of these parameters relative to the parameter in the absence of the test molecule indicates that the test molecule is an agonist or an antagonist of the complex. Methods for identifying such agonists or antagonists may be carried out using the modified yeast two-hybrid assay described herein, or may be carried out *in vitro,* for example.

Test agents to be screened for antagonist or agonist activity may be provided as mixtures of specified compounds, or as compound libraries, peptide libraries, antisera, antisense nucleic acids, random or combinatorial libraries, chemically synthesized libraries, recombinant libraries, or *in vitro* translation-based libraries. Agents may be screened for activity as competitive or non-competitive inhibitors of HR-IP interaction. In particular, fragments or analogs of HR or the IP may be screened for such inhibitory activity. Agents may be screened for inhibition or enhancement of binding of HR and IP under aqueous or physiological binding conditions in which HR-IP binding occurs in the absence of the agent to be tested. Agents that lessen the binding are identified as antagonists of the complex, and agents that enhance the binding are identified as agonists of the complex.

Typical binding conditions are, for example, an aqueous solution of 10-250 mM NaCl, 5-50 mM Tris-HC!, pH 5-8, 0.5% Triton X-100. Metal chelators or divalent cations may be added to improve binding. Binding temperatures may include that of an ice bath up to 42 degrees C. The time of incubation is sufficient to allow binding equilibrium to occur. Methods known to one of skill in the art in light of the present disclosure may be used to assay for binding between HR and an interacting partner and a test agent, including use of detectable labels and quantification of complex formation.

A yeast two-hybrid assay system may be used to test an agent for antagonist or agonist activity for HR-IP binding similar to the assay system set forth by US Patent Number 5,986,055, issued November 16, 1999, for CDK2-interacting proteins, incorporated herein by reference. The two-hybrid assay is carried out as described in the present examples, for the exception that it is done in the presence of a test agent. An increase or decrease in reporter gene activity relative to the activity exhibited in the absence of the test agent indicates that the test agent has an effect on the interacting pair.

Components of such a system include a reconstitutable transcriptional regulator and a reporter gene. The reconstitutable transcriptional activator comprises a DNA binding domain (BD) and an activator domain (AD) that, when reconstituted, induces transcription of the reporter gene. The nucleotide sequence encoding HRt, fragment, derivative, or analog thereof is fused to either the DNA binding domain or the activator domain, and the nucleotide sequences encoding an interacting partner identified from the data of Table 1 is fused to the other of the BD or AD. The DNA binding domain can be any DNA binding domain, as long as it specifically recognizes a DNA sequence within a promoter active for a reporter gene. The activator domain has activity for the binding domain of the transcriptional regulator. The reporter gene is operably linked to a promoter that contains a binding site for the DNA binding domain. As for example, binding of BD-HR to AD-IP leads to reconstitution of the BD-AD transcriptional regulator that activates expression of the reporter gene. The activation of transcription of the reporter gene occurs intracellularly, e.g., in prokaryotic or eukaryotic cells, preferably in cell culture. The reporter gene encodes a detectable marker molecule that can give rise to a detectable signal, *e.g.* a fluorescent protein or a protein that can be readily visualized or that is recognizable by a specific antibody or an enzyme such as LacZ or $\alpha$-galactosidase and is readily assayed.

Strains of yeast are chosen for selectable markers that confer ability to grow under conditions that do not support the growth of cells not expressing the selectable marker, *e.g.* the selectable marker is an enzyme that provides an essential nutrient and the cell in which the interaction assay occurs is deficient in the enzyme and the selection medium lacks such nutrient. The reporter gene is under the control of the native promoter that naturally contains a binding site for the DNA binding protein, or under the control of a heterologous or synthetic promoter.

Examples of transcriptional regulator proteins that have separable binding and transcriptional activation domains include, for example, the GAL4, the GCN4, and the ARD1 proteins of *S. cerevisiae* and the human estrogen receptor. The DNA binding domain and activation domain that are employed in the fusion proteins need not be from the same transcriptional regulator. For example, a GAL4 or a LEXA DNA binding domain is employed, or a GAL4 or herpes simplex virus VP16 activation domain is employed. In one embodiment, the yeast transcription factor GAL4 is reconstituted by the protein-protein interaction and the host strain is mutant for GAL4. Other embodiments are known to one of skill in the art in light of the present disclosure.

To facilitate isolation of the encoded interacting partners, the fusion constructs can further contain sequences encoding affinity tags such as glutathione-S-transferase or maltose-binding protein or an epitope of an HR or HRt antibody, for affinity purification for binding to the HR-IP. Where the IP is a nucleic acid, an affinity tag may be a hybridizing nucleotide sequence complementary to the nucleic acid IP.

The host cell in which the interaction assay occurs can be any cell, prokaryotic or eukaryotic, in which transcription of the reporter gene can occur and be detected, including but not limited to mammalian (e.g., monkey, chicken, mouse, rat, human, bovine), bacteria, and insect cells, and is preferably a yeast cell. Expression constructs encoding and capable of expressing the binding domain fusion proteins, the transcriptional activation domain fusion proteins, and the reporter gene product are provided within the host cell, by mating of cells containing the expression constructs, or by cell fusion, transformation, electroporation, or microinjection, for example. The host cell used should not express an endogenous transcription factor that binds to the same DNA site as that recognized by the DNA binding domain fusion population. In addition, the host cell is mutant or otherwise lacking in an endogenous, functional form of the reporter gene used in the assay.

Various vectors and host strains for expression of the two fusion protein populations in yeast are known and can be used (see US Patent Number 5,986,055, issued November 16, 1999). For example, yeast strains or derivative strains made therefrom, which can be used are N105, N106, N1051, N1061, and YULH, Y190: MATa, ura3-52, his3-200, lys2-801, ade2-101, trpl-901, leu2-3,112, gal4.DELTA., gal80.DELTA., cyh.sup.r 2, LYS2::GAL1.sub.UAS -HIS3.sub.TATA HIS3, URA3::GAL1.sub.UAS -GAL1.sub.TATA -lacZ. CG-1945: MATa, ura3-52, his3-200, lys2-801, ade2-101, trpl-901, leu2-3,112, gal4-542, ga180-538, cyh.sup.r 2, LYS2::GAL1.sub.UAS -HIS3.sub.TATA HIS3, URA3:: GAL1.sub.UAS17mers(x3) - CYC1.sub.TATA -lacZ. Y187: MAT-.alpha., ura3-52, his3-200, ade2-101, trp1-901, leu2-3,112, gal4.DELTA., gal80.DELTA., URA3::GAL1.sub.UAS -GAL1.sub.TATA -lacZ. SFY526: MATa, ura3-52, his3-200, lys2-801, ade2-101, trp1-901, leu2-3,112, gal4-542, gal80-538, can.sup.r, URA3::GAL1-lacZ. HF7c: MATa, ura3-52, his3-200, lys2-801, ade2-101, trp1-901, leu2-3,112, gal4-542, ga180-538, LYS2::GAL1-HIS3. URA3:: GAL1.sub.UAS17MERS(X3) -CYC1-lacZ.. YRG-2: MATa, ura3-52, his3-200, lys2-801, ade2-101, trp1-901, leu2-3,112, gal4-542, ga180-538 LYS2::GAL1.sub.UAS - GAL1.sub.TATA -HIS3, URA3::GAL1.sub.UAS17mers(x3) -CYC1-lacZ.

Plasmids are capable of autonomous replication in a host yeast cell and preferably are capable of being propagated in *E. coli*. The plasmid contains a promoter directing the transcription of the DNA binding or activation domain fusion genes, and a transcriptional termination signal. The plasmid also preferably contains a selectable marker gene, permitting selection of cells containing the plasmid. The plasmid can be single-copy or multi-copy. Single-copy yeast plasmids that have the yeast centromere may also be used to express the activation and DNA binding domain fusions. In another embodiment, the fusion constructs may be introduced directly into the yeast chromosome via homologous recombination using methods known to one of skill in the art in light of the present disclosure.

Plasmids encoding the separate fusion proteins can be introduced into a single host cell (e.g., a haploid yeast cell) containing one or more reporter genes, by co-transformation, to conduct the assay for HRt-IP interactions. Alternatively, the two fusion proteins are introduced into a single cell by mating (e.g. for yeast cells) or cell fusions (e.g., of mammalian cells). In a mating type assay, conjugation of haploid yeast cells of opposite mating type that have been transformed with a binding domain fusion expression construct and an activation domain fusion expression construct, respectively, delivers both constructs into the same diploid cell. The mating type of a yeast strain may be manipulated by transformation with the HO gene.

In one aspect, a yeast interaction mating assay is employed, using two different types of host cells, strain-types a and alpha, of the yeast *Saccharomyces cerevisiae*. One set of host cells, for example the a strain cells, contains fusions of the HRt nucleotide sequences with the DNA-binding domain of a transcriptional activator, such as GAL4. The hybrid proteins expressed in this set of host cells are capable of recognizing the DNA-binding site on the reporter gene. The second set of yeast host cells, for example alpha strain calls, contains nucleotide sequences as identified by the data provided in Table 1 fused to the activation domain of a transcriptional activator. In one embodiment, the fusion protein constructs are introduced into the host cell as a set of plasmids.

Bacteriophage vectors may be used to express the DNA binding domain and/or activation domain fusion proteins. Libraries can generally be prepared faster and more easily from bacteriophage vectors than from plasmid vectors.

Reporter genes include URA3, HIS3, lacZ, MEL1, GFP, luciferase, LEU2, LYS2, ADE2, TRP1, CAN1, CYH2, GUS, CUP1, or CAT, for example. Expression of LEU2, LYS2, ADE2 and TRP1 are detected by growth in a specific defined media; GUS, lacZ, MEL1, and CAT can be monitored by well known enzyme assays; and CAN1 and CYH2 are detected by selection in the presence of canavanine and cycloheximide. The natural fluorescence of GFP is detected. In another embodiment, transcription of the reporter gene is detected by a linked replication assay known to those of skill in the art in light of the present disclosure. In another embodiment, the expression of a reporter gene is detected by immunoassay. Activity of the reporter gene lacZ is monitored by measuring a detectable signal, such as X-gal (5-bromo-4-chloro-3-indolyl-.alpha.-D-galactoside).

False positives arising from transcriptional activation by the DNA binding domain fusion proteins in the absence of a transcriptional activator domain fusion protein are prevented or reduced by negative selection.

In one embodiment of the invention, the DNA sequences encoding the pairs of interacting partners are isolated by amplification in separate respective reactions. Amplification is carried out by polymerase chain reaction known by one of skill in the art using pairs of oligonucleotide primers specific for either the DNA-binding domain hybrids or the activation domain hybrids. Other amplification methods known in the art can be used, including but not limited to ligase chain reaction, use of Q beta. replicase, and the like.

*DUALhybrid Screen II Package B Screening*

The DUALhybrid Screen II Package B System, available from Dualsystems Biotech AG of Zurich, Switzerland, was used to identify interaction partners for HR in human keratinocytes. A full description of this screening method is available from Dualsystems Biotech, available by submitting an electronic mail request at info@dualsystems.com or via the Internet at http://www.dualsystems.com.This screening method generally involves the steps of constructing and verifying a bait vector; transforming a large-scale library and selecting positive clones; isolating and restricting the analysis of positive clones; retransforming and testing clones for bait dependency; and sequencing bait dependent clones. Information on the precise vector library (pACT2), as well as the complete nucleotide sequence employed is available at the web site of Clontech, http://www.clontech.com.

*Purification of HR-IP complexes*

HR-IP complexes may be isolated and purified by standard methods known in the art from natural sources or recombinant host cells expressing the complexes, including but not limited to column chromatography (*e.g.*, ion exchange, affinity, gel exclusion, reverse-phase high pressure, fast protein liquid, and the like), differential centrifugation, differential solubility, or by other standard techniques used in purification of protein complexes or protein-nucleic acid complexes. The amino acid sequence of an interacting protein can be deduced from the nucleic acid sequence of the chimeric gene from which it was encoded. As a result, the protein or a derivative thereof can be synthesized by standard chemical or recombinant methods known in the art.

*Third Aspect: Formulations and Products Comprising Agonist and/or Antagonist Compounds of HR-IP and Administration Thereof*

In yet another aspect of the present invention, compounds demonstrating agonist and/or antagonist activity toward the hairless protein interaction complex, may be formulated into a composition or product for the conveyance of one or more material beautification and/or improvement benefits to mammalian skin. The concentration of an antagonist active or an agonist active, identified by methods of the present invention, in a composition may be varied over a wide range up to a saturated solution, preferably from 0.01% to 99% by weight. The maximum amount effectively applied is limited by the rate at which the active penetrates the skin. Generally, the effective amount ranges from 100 micrograms to 3000 micrograms per square centimeter of skin. The composition may be applied once or twice a day, or even more frequently, for a period of time of 2 weeks to 6 months to achieve a perceived effect upon the beautification and/or improvement of mammalian skin.

The composition may be administered topically by any convenient means. Topical compositions that can be applied locally to the skin may be in a form such as a solution, an oil, cream, ointment, gel, lotion, shampoo, leave-on and rinse-out hair conditioner, milk, cleanser, moisturizer, spray, skin patch, or the like. The composition may include a non-toxic dermatologically acceptable vehicle or carrier that is adapted to be spread on the skin. Examples of suitable vehicles are acetone, alcohols, or a cream, lotion or gel that can effectively deliver the active compound. In addition, a penetration enhancer may be added to the vehicle to further enhance the effectiveness of the formulation.

The antagonist active or agonist active compound-containing compositions and products of the present invention should be applied to an area of mammalian, and particularly human, skin for which the conveyance of material beautification and/or improvement benefits is desired. For example, a woman may choose to apply a HR-IP agonist-containing composition to a portion of her face, arms, neck, or any other region of skin for which material beautification and/or improvement is desired. Similarly, a man may increase the health and vitality of his skin by applying such a composition to an area in need of treatment or for which treatment is desired. The beautification and/or improvement of mammalian skin is demonstrated when any material beautification and/or improvement benefit, as defined hereinbefore, is perceived.

One of ordinary skill in the art would be able to select appropriate adjunct ingredients and amounts to formulate in the compositions described above without undue experimentation, depending on the antagonist active or an agonist active selected, the intended use of the resulting composition and the needs and/or abilities of the formulator. Indeed, the combined and systematic use of products and formulations of the present invention serves beautify and improve the condition of the mammalian skin, and particularly human skin, to which they are applied. In accordance with this aspect of the present invention, said products and formulations will take a variety of shapes and forms, depending on the specific needs and/or abilities of the practitioner of the present invention, as well as the purpose for which their employment is sought. In any instance, it is believed that use of the compositions in accordance with the present invention results a marked reduction to fine lines and wrinkles, as well as an improvement in the overall appearance of mammalian, and particularly human, skin.

Moreover, the amount and/or ratio of antagonist active and/or an agonist active compounds incorporated into the products and formulations of the present invention will depend on the purpose for which employment of the subject product and/or formulation is desired. Nevertheless, in one aspect of the present invention, the products and formulations disclosed herein will comprise from about 0.0001% to about 10%, preferably from 0.05% to about 10%, more preferably from about 0.1% to about 5%, most preferably from about 0.5% to about 3% of either a antagonist active or an agonist active compound. In yet another aspect of the present invention, the products and formulations disclosed herein will comprise a combination of both an antagonist active or an agonist active compound. In such an instance, the present products and formulations comprise from about 0.05% to 20%, preferably from about 0.1% to about 3%, of a antagonist active compound and from about 0.01% to about 5%, preferably from about 0.05% to about 1%, of an agonist active compound.

In accordance with another aspect of the present invention, personal care products comprising the antagonist active or agonist active compounds of the present invention are disclosed. Suitable but non-limiting product forms include emulsions, gels, lotions, creams, ointments, mousses, sprays, mists, sticks, powders; wipes and combinations thereof. Suitable personal care products comprising antagonist active or agonist active compounds, include, but certainly are not limited to: hand soaps, hand sanitizers, body washes, mouth washes, toothpastes, shower gels, shampoos, hair conditioners, hand and/or body lotions, facial lotions, facial creams, foundations, lip sticks, rouges, deodorants and combinations thereof. In yet still another aspect of the present invention, the antagonist active or agonist active compounds of the present invention are incorporated into one or more household care products. Indeed, suitable household care products for purposes of the present invention include, but are not limited to: hard surface cleaners, deodorizers, fabric care compositions, fabric cleaning compositions, manual dish detergents, automatic dish detergents, floor care compositions, kitchen cleaners or disinfectants, bathroom cleaners or disinfectants and combinations thereof.

In another aspect of the present invention, the antagonist active or agonist active compounds of the present invention are incorporated, both alone and/or in combination into a skin care product. In one aspect of the present invention, the skin care product incorporates a dermatologically acceptable carrier to facilitate safe transfer of the present compounds to a desired area of mammalian skin. In another aspect of the present invention, the skin care product of the present invention comprises certain adjunct ingredients. Said adjuncts include, but certainly are not limited to: antimicrobial and antifungal actives, surfactants, desquamation actives, anti-acne actives, anti-wrinkle actives, anti-atrophy actives, anti-oxidants, radical scavengers, chelators, flavonoids, anti-inflammatory agents, anti-cellulite agents, topical anesthetics, tanning actives, sunscreen actives, conditioning agents, thickening agents, detackifying agents, odor control agents, skin sensates, antiperspirants and mixtures thereof. Indeed, a complete description and examples of each of the aforementioned adjunct ingredients is set forth in US Patent Number 6,294,186, assigned to The Procter and Gamble Company, Cincinnati, Ohio and incorporated herein by reference. In another aspect of the present invention, the compounds disclosed herein are incorporated into a woven or non-woven wipe form of a skin care product, particularly such a product marketed by The Procter and Gamble Company of Cincinnati, Ohio. In yet another aspect of the present invention, the antagonist active or agonist active compounds disclosed herein are incorporated into a topical skin care product, including but not limited to, lotions, creams, gels and ointments, particularly those marketed by The Procter and Gamble Company of Cincinnati, Ohio. Indeed, the precise form of suitable skin care products into which the antagonist active or agonist active compounds of the present invention may be incorporated will depend on the needs and/or abilities of the formulator of said products.

*Administration of Compounds, Compositions and Products*

In yet another aspect of the present invention, the antagonist active or agonist active compounds disclosed herein are formulated into compositions and/or products for administration onto mammalian, and particularly human, skin. In one aspect of the present invention the topical formulations disclosed herein include a safe and effective amount of beautification and/or improvement agents and other ingredients that are adapted to enhance the appearance of the mammalian skin onto which they are applied.

By "safe and effective amount", it is intended that an incorporated amount of a compound or composition be high enough to significantly improve the appearance of the skin, but low enough to discourage side effects, which may actually reduce the appearance and beauty of the skin. Indeed, the safe and effective amount of an agent for use in the compounds and/or compositions of the present invention will vary depending on one or more of the following factors: the nature of the skin for which treatment is sought, the age and physical condition of the skin for which treatment is sought, the severity of any existing skin conditions, the intended duration of the treatment, the existence and nature of any concurrent therapy, the particular agent for which employment is sought, the particular excipients utilized, and the needs and/or abilities of the formulator of the present compounds and compositions. Nevertheless, the appropriate amount of the agent, preferably the antagonist active or agonist active compounds disclosed herein, to be incorporated into the present compositions may be determined by routine experimentation with animal models. Indeed, one such model includes, but certainly is not limited to, intact and aged murine models of mammalian, and particularly human, skin.

The antagonist active or agonist active compounds of the present invention may be administered systemically, *e.g.*, orally and/or parenterally, including subcutaneous or intravenous injection, and/or intranasally, but especially transdermally. In one aspect of the present invention, the antagonist active or agonist active compounds disclosed herein are applied directly to the mammalian skin for which treatment is sought in a unit dosage form. The precise amount of the present compounds incorporated into a unit dosage form will depend upon one or more factors disclosed hereinbefore, and particularly the needs and/or abilities of the formulator of the present compositions and the nature of the mammalian skin for which treatment is desired.

In yet another aspect of the present invention, the dose forms for use with the present compounds and compositions include nasal, transdermal, rectal, sublingual, oral and combinations thereof. In another aspect of the present invention, one or more carriers suitable for use in the present invention may be employed to achieve delivery of the present compounds and compositions, and particularly for injection or surgical implants. Said carriers include, but certainly are not limited to: hydrogels, controlled- or sustained release devises, polylactic acid, collagen matrices, and combinations thereof. In another aspect of the present invention, implant devices are coated with the antagonist active or agonist active compounds and/or formulations disclosed herein. In another aspect of the present invention, the antagonist active or agonist active compounds and/or formulations disclosed herein are dissolved in a buffer and mixed with a collagen gel for coating onto the porous end of an implant device.

Indeed, in a further aspect of the present invention, the compounds disclosed herein are administered orally. In this respect, oral forms suitable for administration of the present compounds and formulations include, but certainly are not limited to: liposomes, lipid emulsions, proteinaceous cages, other excipients and combinations thereof. Use of the term "excipients" herein is intended to encompass any physiologically inert, pharmacologically inactive material known to those of ordinary skill in the art. Suitable excipients for use in the present invention are compatible with the physical and chemical characteristics of the particular antagonist active or agonist active ingredient for which employment is sought, as well as the mammalian skin substrate for which application is desired. In one aspect of the present invention, suitable excipients for use herein include, but are not limited to, polymers, resins, plasticizers, fillers, lubricants, binders, disintegrants, solvents, co-solvents, buffer systems, surfactants, preservatives, sweetening agents, flavoring agents, fragrance agents pharmaceutical grade dyes, pigments and combinations thereof.

*Articles of Manufacture*

In another aspect of the present invention, articles of manufacture comprising the antagonist active or agonist active compounds of the present invention and/or one or more of the aforementioned products, are intended for personal care, skin care and household care applications. The article of manufacture of the present invention encompasses one or more products as described hereinbefore that may be packaged in a container or dispenser with a set of instructions for the consumer. The article of manufacture of the present invention typically comprises (a) container or dispenser, (b) product and (c) set of instructions to apply said product to an appropriate substrate to convey material beautification and improvement benefits to mammalian skin. Containers and/or dispensers suitable for the article of manufacture of the present invention include, but are not limited to: PET bottles and tubs, flow-wrap pouches, foaming dispensers, spray dispensers and combinations thereof. To reiterate, the article of manufacture of the present invention further comprises a set of instructions in association with the container. By "in association with," it is meant that the instructions are either directly printed on the container or dispenser itself or presented in a different fashion including, but not limited to: a brochure, print advertisement, electronic advertisement and/or verbal communication, so as to communicate the set of the instructions to a consumer of the article of manufacture.

The set of instructions typically comprise the instructions relating to the use of the product to apply the antagonist active or agonist active compounds of the present invention onto a suitable substrate for which treatment is sought. The set of instructions may further comprise the instruction to allow the present compounds to remain on the treated substrate, without rinsing or otherwise removing the compounds from the treated substrate. Nevertheless, the precise instructions included with the article of manufacture of the present invention will depend on the specific compounds and the product for which the inclusion of instructions is desired and the substrate onto which application of the product is intended. In another aspect of the present invention, the instructions included ir the present articles of manufacture reflect the methods disclosed and claimed herein.

*Fourth Aspect: Methods of Using Compounds, Compositions and Products*

In yet another aspect of the present invention, methods of using the claimed compounds, compositions and products to convey one or more material beautification and/or improvement benefits to mammalian skin are disclosed. In one aspect of the present invention, a method for improving and/or beautifying mammalian skin is disclosed. Said method comprises the steps of delivering one or more agonist and/or antagonist compounds to a portion of mammalian skin for which improvement and/or beautification is desired, and optionally, removing said compounds following their delivery (if administered topically). In another aspect of the present invention, a method of administering a composition and/or product comprising the agonist and/or antagonist compounds of the present invention is disclosed. Said method comprises the steps of delivering said composition or product to a portion of mammalian skin for which beautification and/or improvement is desired, and optionally, removing said composition or product following its delivery (if administered topically). Indeed, the systematic use of the methods disclosed herein conveys one or more material, beautification and/or improvement benefits to the mammalian, and particularly human, skin on which the methods disclosed herein are used.

In yet another aspect of the present invention, a method of treating epidermal, mammalian skin using the compounds, compositions and/or products described herein, is disclosed. Said method generally involves the steps of administering the compounds, compositions and/or products disclosed herein to a portion of epidermal mammalian skin for which beautification and/or improvement is desired, and optionally, removing said compounds, compositions and/or products following their delivery (if administered topically).

In yet still another aspect of the present invention, a method for beautifying and/or improving mammalian skin, whether epidermal or otherwise, using a human sphingolipid activator protein is disclosed. Said method comprises the steps of delivering the human sphingolipid activator protein to an area of mammalian skin for which beautification and/or improvement is desired, and optionally, removing said compound following its delivery (in the case of topical administration). It should be noted that the present inventors have previously disclosed that the human sphingolipid activator protein, associated with the GeneBank Accession Number M60255, to be an interacting partner of hairless protein that inhibits and/or stimulates hair growth. However, never hereinbefore has the human sphingolipid activator protein been disclosed to be useful in the beautification and/or improvement of mammalian skin.

In accordance with yet another aspect of the present invention, a method of controlling weight gain and/or loss using the compounds, compositions and/or products of the present invention is disclosed. Said method, too, generally involves the steps of delivering the present compounds, compositions and/or products to an area of a mammal for which weight control is desired, and optionally, removing said compounds, compositions and/or products following their delivery (if administered topically). Without wishing to be bound by theory, it is believed that the hairless protein interacting complexes of the present invention, and in particular LXR, affect mammalian weight gain and loss. This belief is based upon published literature indicating that hairless protein is also expressed in adipose and its own expression is down regulated during development of hyperglycemia. See Nadler et al (2000); The Expression of adipogenic genes is decreased in obesity and diabetes mellitus, Proc Natl Acad Sci (USA) 97, 11371-11376. Further, other literature has disclosed that LXR is abundantly expressed in adipose and is implicated in control of adipogenesis. Indeed, LXR mediates upregulation of lipogenic enzymes (including acetyl CoA carboxylase, fatty acid synthetase, Stearoyl-CoA desaturase and etc.), through its ability to upregulate the steroid regulatory element binding protein (SREBP). LXR has also been disclosed to upregulate lipoprotein lipase expression. See Also Schultz JR, et al., *Role of LXRs in control of Lipogenesis*, Tularik Inc., South San Francisco, California 94080 (USA).

Other publications have disclosed that the discovery of oxysterols as the endogenous liver X receptor (LXR) ligands and subsequent gene targeting studies in mice provided strong evidence that LXR plays a central role in cholesterol metabolism. The identification here of a synthetic, nonsteroidal LXR-selective agonist series represented by T0314407 and T0901317 revealed a novel physiological role of LXR. Oral administration of T0901317 to mice and hamsters showed that LXR activated the coordinate expression of major fatty acid biosynthetic genes (lipogenesis) and increased plasma triglyceride and phospholipid levels in both species. Complementary studies in cell culture and animals suggested that the increase in plasma lipids occurs via LXR-mediated induction of the sterol regulatory element-binding protein 1 (SREBP-1) lipogenic program. See Stulnig et al. 2002, Novel roles of liver X receptors exposed by gene expression profiling in liver and adipose tissue, Mol Pharmacol. 62, 1299-1305. Stulnig et al.2002, Liver X receptors downregulate 11beta-hydroxysteroid dehydrogenase type 1 expression and activity, Diabetes 51, 2426-2433. Ross et al. 2002, Microarray analyses during adipogenesis: understanding the effects of Wnt signaling on adipogenesis and the roles of liver X receptor alpha in adipocyte metabolism, Mol Cell Biol 22, 5989-5999. Matsuzaka et al. 2002, Cloning and characterization of a mammalian fatty acyl-CoA elongase as a lipogenic enzyme regulated by SREBPs, J Lipid Res 43, 911-920. Zhang et al. 2001, Regulation of lipoprotein lipase by the oxysterol receptors, LXRalpha and LXRbeta, J Biol Chem 276, 43018-43024. Yoshikawa et al. 2001, Identification of liver X receptor-retinoid X receptor as an activator of the sterol regulatory element-binding protein 1c gene promoter, Mol Cell Biol 21, 2991-3000. Laffitte et al. 2001, LXRs control lipid-inducible expression of the apolipoprotein E gene in macrophages and adipocytes, Proc Natl Acad Sci U S A 98, 507-512.

In yet another aspect of the present invention, a method of treating diabetes using the compounds, compositions and products of the present invention is disclosed. Said method comprises the steps of administering the compounds, compositions and products of the present invention to a mammal in need of diabetes treatment, and optionally, removing said compounds, composition and/or products following their delivery (if administered topically).

PREPARATIVE EXAMPLES

EXAMPLE 1 - *Interaction Partners (IP's) of Hairless Protein*

The present example provides for Hairless Protein (HR) interacting partners (HR-IP) using the yeast two-hybrid technology. HR cDNA corresponding to the C-terminal half of HR protein (aa residues 490 to 1182, hereinafter HRt) is cloned into DUALhybrid bait vector so as to express Lex A DNA BD-HRt in yeast. Yeast cells expressing BD-HRt are transformed with human keratinocyte cDNA library in the activation domain (AD) vector. The resultant interacting partners provided by the present invention are listed in Table 1 that include molecules involved in cell cycle, cell differentiation, transcription, nuclear transport, signal transduction, cellular integrity, and mitochondrial machinery. Thus, HRt (and HR) appears to be a well-connected, multi-functional protein.

*Materials*

A mouse early anagen cDNA library is constructed by personnel at Procter & Gamble using standard techniques. *E. coli* expression plasmid pET32a (Catalog No. 69015-3; component plasmid of Novagen's pET prokaryotic expression system) and *E. coli* strain BL21(DE3) competent cells (Catalog No. 69387-3) are purchased from Novagen (Madison, WI). Custom designed oligonucleotides are synthesized by personnel at Procter & Gamble using standard techniques or made by BD Biosciences Clontech (PaloAlto, CA). DNA sequence determination is performed by personnel at Procter & Gamble using standard techniques or contracted out to Seqwright Inc (Houston, TX). DNA sequence analyses and data base searches for homology are performed using SeqWeb version 1.2 (in conjunction with Wisconsin Package Version 10.1). Various microbiological media are purchased from BD Biosciences Clontech (PaloAlto, CA) and DIFCO Becton Dickinson Microbiology Systems (Sparks, MD). Various pre-made media plates for cultivation of *E. coli* and yeast are purchased from Gibson Laboratories (Lexington, KY). Matchmaker two-hybrid System 3 (Catalog No. K1612-1), pre-transformed human brain Matchmaker cDNA library in pACT2 vector (Catalog No.HY4004AH, Lot No.9070550), Advantage 2 PCR kit (catalog No. K1910-1), and Matchmaker AD LD-insert screening amplimer sets (Catalog No. 9103-1), are purchased from BD Biosciences Clontech (PaloAlto, CA). Male mice (C3H strain) are purchased from Harlan Labs (Indianapolis, IN). NAIR lotion hair remover (4 minute formula) is purchased from a local Walgreens store. Dolgel Gel (5% ibuprofen) is purchased from Life Extension International Center Inc. (Coral Gables, FL). Restriction enzymes, T4 DNA ligase, molecular weight markers, agarose, and other routine molecular biology reagents are purchased from Life Technologies (Gaithersburg, MD).

*Cloning and characterization of mouse Hairless (HR) cDNA*

Oligonucleotides are designed to PCR-amplify the desired portion of mouse HR cDNA, based on the published sequence for mouse HR "Structure and Expression of the Hairless Gene of Mice," Begona, M., et al., J. Proc. Natl. Acad. Sci, USA 91:7717-7721, 1994) (GenBank accession no. Z32675). The oligonucleotides are also designed to contain restriction enzyme recognition sites for *Eco* RI, *Xba* I, *Not* I and *Bam* HI restriction enzymes for subsequent manipulation of the PCR product. In particular, the restriction enzyme sites for *Eco* RI and *Not* I (noted as underlined and italicized in the oligonucleotides shown below) are particularly useful for subsequent cloning and manipulation. The forward and reverse oligonucleotide primer pairs 5'-CCG GAA TTC GTC ACC CAG TGC CAA AGC TGT (SEQ ID NO:100) and 5'-CGG GAT CCT CTA GAG CGG *CCG CTT* ATT ATT TAG CTT CCT GTA ACG CCCC (SEQ ID NO:101) are used to PCR amplify HR cDNA corresponding to nucleotides 1845-3923 of HR from mouse early anagen cDNA library. PCR amplification is performed using the standard PCR protocol supplied with the Advantage 2 PCR kit. The PCR product is analyzed by agarose gel electrophoresis and is found to have the expected size (approximately 2 Kb). The PCR product is excised with *Eco* RI and *Not* I restriction enzymes. The restricted product is gel-purified and inserted into *Eco* RI and *Not* I sites of pET32a, such that the insert makes an in-frame fusion with upstream sequences. The resulting plasmid is designated pET32a-HRt. This plasmid is used for both nucleotide sequence confirmation as well as for protein expression in *E. coli.* The nucleotide sequence reveals changes at three positions (nucleotide position 2166, change from C to T causing a codon change from AGC to AGT; nucleotide position 2611, change from C to T causing a codon change from GAC to GAT; nucleotide position 2916, change from T to C causing a codon change from CCT to CCC) from the published sequence (SEQ ID NO:102). Amino acid number 1 of SEQ ID NO:103 corresponds to amino acid residue 490 of the C-terminal portion of HR protein. This truncated HR (HRt) cDNA corresponds to amino acid residues 490-1182 of the C-terminal portion of HR protein.

However, none of the above-cited changes results in an amino acid change. In essence the HRt cloned herein is predicted to code for a protein product identical to that of mouse HRt. The zinc finger region (amino acids 595-620) and three PEST domains are present in the 490-1182 amino acid portion of HR. The PEST domains (region 522-546 having a score of 12.26, region 709-722 having a score of 7.56, and region 729-744 having a score of 21.71) are expected by the present inventors to be important in HR function. Mouse HR is greater than 80% identical to human HR. PEST sequences are often found to serve as signals for rapid protein degradation. PEST sequences are constitutive or conditional signals. Constitutive means "as such," while conditional means "upon modification" (such as phosphorylation, *cis-trans* isomerisation induced by light, ligand binding, etc.). To objectively determine whether a protein contains a PEST region, a computer program called PEST-FIND has been developed by M. Rechsteiner and S. W. Rogers (PEST Sequences and Regulation by Proteolysis, TIBS 21, July 1996). The algorithm, which is available in PC/ GENE (OMIGA), defines a PEST sequence as a hydrophilic stretch of 12 or more amino acid residues containing at least one each of Pro, Glu/Asp, and Ser/Thr in any order or combination. They also have to be flanked by basic amino acid residues Lys, Ala, or His, but basic residues are disallowed within the PEST sequence. PEST-FIND produces a score ranging from about +50 to -50. By definition, a score >0 denotes a possible PEST region, but a value > +5 indicates a highly probable PEST region. The PEST regions noted herein also have numerous potential protein kinase C and casein kinase II phosphorylation sites. The PEST scores cited supra are among the best ever reported in the literature.

pET32a-HRt is also an inducible *E. coli* expression plasmid. Protein expression is studied using the standard protocol for *E. coli* expression as also provided by the supplier. SDS-PAGE (10-20%) analysis of total protein isolated from quadruplicate *E. coli* BL21(DE3)/pET32a-HRt clones induced for protein expression reveals a high level of expression of protein product of the expected size for a HRt fusion protein just after 2h of induction. Control strain *E. coli* BL21 (DE3)/ pET32a does not show induction of any protein expression at the high molecular weight region as compared to the *E. coli* BL21(DE3)/ pET32a-HRt clones. This study confirms expression of protein product from the HRt insert. The protein product is not recoverable under non-denaturing conditions.

*Cloning of HRt into GAL4 binding domain (BD) yeast two-hybrid (Y2H) vector*

The HRt insert from pET32a-HRt is excised as an *Eco* RI-*Xho* I fragment (there is a unique *Xho* I site just adjacent to HRt insert) and inserted at the *Eco* RI-*Sal* I sites of Y2H-BD vector pGBKT7 (Catalog No. K1612-B; a component of Matchmaker two-hybrid System 3 (Catalog No. K1612-1) BD Biosciences Clontech, Palo Alto, CA) to obtain pGBKT7-HRt in which HRt is fused in-frame to GAL4 DNA BD (this plasmid is also designated GAL4 BD-HRt). This plasmid was combined with AD-IP in *S. cerevisiae* AH109 strain screen for agonists and antagonists of HRt-IP interaction (see Example 2 and 3). However, to screen for IP in the first place, HRt from GAL BD-HRt was excised out and inserted into DUALhybrid bait vector (this plasmid is also designated LexA BD-HRt).

*Human Keratinocyte cDNA Library Screen for HRt interaction*

Clontech Human Keratinocyte GAL4 cDNA library (complexity $2.5 \times 10^6$) in pACT2 vector was used used to screen for HRt interacting proteins. A Leu⁻, Trp⁻ yeast strain with HIS3 and LACZ reporters for yeasts two hybrid interaction (strain L41) was first transformed with LexA BD-HRt palsmid) for Trp⁺ phenotype. The resulting L41/LexA BD-HR transformant was screened to ensure expression of LexA BD-HRt protein and also for lack of auto activation of the reporter genes HIS3 and LACZ. This strain was transformed with Keratinocyte AD-cDNA library and plated on Trp⁻Leu⁻His⁻ plate to select for clones with positive interaction. Colonies thatappeared on selsctive plates were subsequently tested for LACZ expression on X-GAL plates. A total of 90 colonies were identified. AD-plasmid was rescued from the 90 clones and retested for HRt bait dependance. Fifty four isolates were confirmed as positive. The positive clones are expected to contain an AD-IP (interacting partner) that interacts with BD-HRt. To characterize IP, the sequence of IP is determined and matched to the human genome database DNA sequence analysis and data base search for homology are performed using SeqWeb version 1.2 (in conjunction with Wisconsin Package Version 10.1).

Several human interacting partners that interact with HRt are identified as listed in Table 1. Proteins having SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO:13, SEQ ID NO:14 and SEQ ID NO: 15 appear to be new proteins.

**Table 1**

*BD-HRt(mouse) interaction with human keratinocyte c-DNA library in AD-Vector *Represents AD-HRt(mouse) interaction with BD-VDR(mouse)*

| GenBankAcc# | Clone Identity | Hits | SEQ ID NO: |
|---|---|---|---|
| BC024292 | Homo Sapiens Keratin 5 | 1 | 1 |
| BC007790 | Homo Sapiens Ubiquitous Receptor | 14 | 2 |

(continued)

*BD-HRt(mouse) interaction with human keratinocyte c-DNA library in AD-Vector *Represents AD-HRt(mouse) interaction with BD-VDR(mouse)*

| GenBankAcc# | Clone Identity | Hits | SEQ ID NO: |
|---|---|---|---|
| AF167160 | Homo Sapiens Protein Inhibitor of Activated STAT-1(PIAS1) | 1 | 3 |
| BC001765 | Homo Sapiens Similar to Stromal Antigen 2 | 4 | 4 |
| XM_113678 | Homo Sapiens Nucleoporin 160 Kda | 3 | 5 |
| M57707 | Homo Sapiens Retinoic Acid Receptor Gamma-1 | 1 | 6 |
| BC000261 | Homo Sapiens Thyroid Hormone Receptor Alpha | 7 | 7 |
| BC001275 | Homo Sapiens Annexin A1 (Lipocortin 1 or P35) | 1 | 8 |
| AF054589 | Homo Sapiens HIC Protein Isoform P32 and Isoform 40 | 1 | 9 |
| BC011708 | Homo Sapiens Insulin-like Growth Factor Binding Domain Protein 6 | 1 | 10 |
| BC002412 | Homo Sapiens Inner Membrane Protein, Mitochondrial (Mitofilin) | 3 | 11 |
| BC001493 | Homo Sapiens Endoplasmic reticulum thioredoxin superfamily member | 1 | 12 |
| BC030556 | Homo Sapiens Protein Inhibitor of Activated STAT-3 (PIAS3) | 1 | 13 |
| BC011819 | Homo Sapiens DEAD (Asp-Glu-Ala-Asp) box polypeptide 3 | 2 | 14 |
| BC015970 | Homo Sapiens Dpy-30 Like Protein (Dev. Nuclear Protein) | 1 | 15 |
| BC006716 | Mus Musculus Vitamin D Receptor (VDR) | 1 | 16 |

EXAMPLE 2 - *Screen for compounds that disrupt BD-HRt><AD-IP interaction in yeast strain*

AH109 was used for compound screening. AH109 (a component of the Matchmaker two-hybrid System 3 (Catalog No. K1612-1) BD Biosciences Clontech, Palo Alto, CA) is a desirable yeast reporter strain for Y2H interaction. It contains *ADE2, HIS3, lacZ* and *MEL1* reporter genes, each of which uses a distinct GAL4-responsive promoter. The various Y2H recombinant plasmids are introduced into the yeast strain AH109 by using a lithium acetate-mediated yeast transformation protocol as provided by the supplier of the Y2H system (BD Biosciences Clontech, PaloAlto, CA). Transformants for combined AD and BD plasmids are selected based on their ability to grow in Leu and Trp double drop out medium (DDO).

Interaction between AD and BD fusion proteins (Y2H interaction) will bring the AD and BD domains of GAL 4 protein close to each other and thus reconstitute a functional GAL 4 protein. This will allow transformants to grow on Leu, Trp, His, Ade quadruple drop out medium (QDO). Growth rate on QDO, as well as a measure of MEL1 (alpha-galactosidase) or lacZ (beta-galactosidase) activities will give a more or less quantitative measure of avidity of Y2H interaction.

Growth inhibition of AH109 (BD-HRt><AD-IP) strain in Leu, Trp, His, Ade QDO, but not on Leu, Trp DDO is used as an initial screen for compounds for disruption of HRt-IP interaction. A diverse library of compounds is screened. Microtiter plates with 200 $\mu$l of QDO and DDO media seeded with freshly cultured yeast cells at 0.01-0.03 optical density at 650 nm (OD650) are treated with various test compounds. Stock solutions of test compounds at 20 mM are made in DMSO and tested at 400 $\mu$M final concentration. The plates are incubated without shaking at 25°C. OD650 is first measured after addition of compounds and recorded for the next 8-10 days. Wells with no compound added (positive control) are expected to reach saturation growth (- 0.7 within 3 days of incubation). Wells with only media and no cells inoculated are expected to remain at the initial OD650 value (-0.03) over the entire period of the assay. Positive compounds from this assay are tested against an unrelated Y2H interaction (p53><T antigen) assay. pGBKT7-53 and pGADT7-T plasmids were used to establish an unrelated (to HRt) Y2H interaction in which p53 protein interacts with T antigen. These plasmids are part of the Matchmaker two-Hybrid system 3(catalog # K1612-1, BD Biosciences Clontech, Palo Alto, CA).

EXAMPLE 3 -. *Screen for compounds that enhance or disrupt BD-HRt><AD-IP interaction*

Increase or decrease in alpha-galactosidase activity of AH109 (BD-HRt><AD-IP) strain grown in Leu, Trp, His TDO in the presence of various test compounds is used to screen for agonists or antagonists of BD-HRt><AD-IP interaction. A diverse library of compounds is screened.
Microtiter plates with TDO media seeded with freshly cultured yeast cells at 0.05 optical density at 650 nm (OD650) are treated with various test compounds at 100-400 uM or vehicle (DMSO) control. Stock solutions of test compounds at 10 - 20 mM are made in DMSO and tested at 100 - 400 $\mu$M final concentration. The final assay mixture comprising of cells, TDO and compound (or DMSO alone) is adjusted to be 100 ul and the amount of DMSO is maintained at or below 2%. The plates are incubated for 3-4 days without shaking at 25°C. Plates are briefly agitated to obtain uniform suspension of cells prior to addition of assay buffer. Assay of reporter enzyme (alpha-galactosidase) activity is performed by incubating for 2-5 hours at 30 degree Centigrade with 100 ul assay buffer [acetate buffer (0.2M, pH 4.5) containing 0.025M para-nitropnenyl alpha galacto pyranoside (PNPG)]. The incubation time can be reduced or increased depending on the extent of alpha galactosidase activity for a given BD-HRt><AD-IP pair. Plates are briefly agitated to obtain uniform suspension of cells and absorbance at both 650 nM $(OD650)_{ref}$ and 410 nm $(OD410)_{ref}$ are measured. $OD650_{ref}$ serves as a reference value for cell density and $OD410_{ref}$ serves as reference value prior to yellow color development for product (p-nitro phenol) generated in the assay. For color development 100 ul of 0.1 M sodium carbonate solution is added and after standing for at least 1 minute, $OD410_{exp}$ is measured. Relative alpha-galactosidase activity is estimated using the following relation:

$$\text{Relative activity} = [OD410_{exp}\text{-}OD410_{ref}\text{x}0.67]/OD650_{ref}$$

Positive compounds from this assay are tested against an unrelated Y2H interaction (p53><T antigen) assay to ensure that the compounds are specific to BD-HRt><AD-IP interaction.

EXAMPLE 4 - *Screen for interaction with AD-HRt*

We have observed that, with certain IPs such as Thyroid hormone receptor (TR), the interaction is much stronger in the AD-HRt><BD-TR configuration than in the BD-HRt><AD-TR configuration. Based on this observation, we suspect that some of the IPs might have gone undetected in the BD-HRt><AD-cDNA library screen and additional IPs for HRt can be detected by also using a AD-HRt><BD-cDNA library screen.
One missed candidate in the BD-HRt><AD-cDNA library screen is Vitamin D receptor (VDR). We speculated that Vitamin D receptor (VDR) may be an IP for HRt, based on the importance of Vitamin D for skin and hair, including the reports that like mutations in the gene encoding HR, mutations in the mammalian VDR gene results in congenital hair loss in both humans and mice (Ref: Vitamin D in Dermatology, Ed. Kragballe.K., Marcel Dekker, Inc., New York, Basel. Pub. 2000). To test this interesting possibility, AD-VDR and BD-VDR constructs were constructed using mouse VDR cDNA (GenBank Acc# BC006716) and tested for interaction with BD-HRt and AD-HRt respectively. As predicted, BD-VDR interacted avidly with AD-HRt. Furthermore, using the assay described in example 3, we were able to show that the interaction was enhanced over 3 fold by VDR ligands 25-hydroxy cholecalciferol (25 HC) (Sigma, H-4014) 1alpha, 25-dihydroxy cholecalciferol (25 DHC) (Sigma, D-1530) tested at submicromolar concentrations. Interestingly, we also found BD-VDR to interact with AD-TRβ and this interaction was enhanced approximately 2 fold by 25 HC and was unaltered by 25 DHC.

All documents cited are, in relevant part, incorporated herein by reference; the citation of any document is not be construed that it is prior art with respect to the present invention. While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A composition comprising a mouse HRt protein-human interacting partner protein complex wherein the human interacting partner protein comprises a molecule selected from the group consisting of: Homo Sapiens Ubiquitous Receptor; Homo Sapiens Retinoic Acid Receptor Gamma 1; Homo Sapiens Protein Inhibitor of Activated STAT-1; Homo Sapiens Similar to Stromal Antigen 2; Homo Sapiens Nucleoporin 160 Kda; Homo Sapiens Thyroid Hormone Receptor Alpha; Homo Sapiens Annexin A1; Homo Sapiens HIC Protein Isoform p32 and Isoform 40; Homo Sapiens Insulin-like Growth Factor Binding Domain Protein 6; Homo Sapiens Inner Membrane Protein Mitochondrial; Homo Sapiens Endoplasmic Reticulum Thioredoxin Super Family Member; Homo Sapiens Protein Inhibitor of Activated STAT-3; Homo Sapiens DEAD Box Polypeptide 3; Homo Sapiens Dpy-30-like Protein; Mus Musculus Vitamin D Receptor; and combinations thereof.

2. A composition according to claim 1, wherein the human interacting partner protein comprises Homo Sapiens Ubiquitous Receptor.

3. A composition according to claim 1, wherein the human interacting partner protein comprises Homo Sapiens Retinoic Acid Receptor Gamma 1.

4. A composition comprising a mouse HRt protein-human interacting partner protein complex, wherein said human interacting partner is encoded by a nucleic acid selected from the group consisting of SEQ ID NO:2, SEQ ID NO:6, SEQ ID NO:3, SEQ ID NO:4, SEQ ID NO:5, SEQ ID NO:7, SEQ ID NO:8, SEQ ID NO:9, SEQ ID NO: 10, SEQ ID NO:11, SEQ ID NO:12, SEQ ID NO: 13, SEQ ID NO:14, SEQ ID NO:15, SEQ ID NO:16 and combinations thereof.

5. A composition according to claim 4, wherein said human interacting partner is encoded by a nucleic acid comprising SEQ ID NO:2.

6. A composition according to claim 4, wherein said human interacting partner is encoded by a nucleic acid comprising SEQ ID NO:6.

7. A method of assaying a test compound for agonist or antagonist activity for the composition of any one of claims 1 to 3, comprising:

   a) measuring a level of interaction between mouse HRt protein and the human interacting partner protein in the absence of the test compound;
   b) measuring a level of interaction between mouse HRt protein and the human interacting partner protein in the presence of the test compound;

   wherein when the level measured in step b) is greater than the level in step a), the test compound has agonist activity, and wherein when the level measured in step b) is less than the level in step a), the test compound has antagonist activity.

8. A method of assaying a test compound for agonist or antagonist activity for the composition of any one of claims 4 to 6, comprising:

a) measuring a level of interaction between mouse HRt protein and the human interacting partner protein in the absence of the test compound;

b) measuring a level of interaction between mouse HRt protein and the human interacting partner protein in the presence of the test compound;

wherein when the level measured in step b) is greater than the level in step a), the test compound has agonist activity, and wherein when the level measured in step b) is less than the level in step a), the test compound has antagonist activity.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6348348 B, Thompson
- WO 9938965 A
- US 45285803 A
- US 5986055 A, Yang, M.
- US 6294186 B

### Non-patent literature cited in the description

- **Ahmad et al.** *Science,* 1998, vol. 279, 720-724
- **Cichon et al.** *Hum. Mol. Genet.,* 1987, vol. 7, 1671-1679
- **Thompson.** *J. Neurosci.,* 1996, vol. 16, 7832-7840
- **Cachon-Gonzalez et al.** *Proc. Natl. Acad. Sci. USA,* 1994, vol. 91, 7717-7721
- **Begona, M. et al.** Structure and Expression of the Hairless Gene of Mice. *J. Proc. Natl. Acad. Sci, USA,* 1994, vol. 91, 7717-7721
- **M. Rechsteiner ; S. W. Rogers.** PEST sequences and regulation by proteolysis. *TIBS,* 21 July 1996
- **Jose et al.** *J Anat,* April 2001, vol. 198, 399-406
- **Stoye et al.** *Cell,* 1988, vol. 54, 383-391
- **Panteleyev et al.** *Exp. Dermatol.,* 1998, vol. 7, 281-288
- **Ahmad et al.** *Genomics,* 1998, vol. 53, 383-386
- **Ahmad et al.** *Exp. Dermatol.,* 1998, vol. 7, 298-301
- **Gonzalez et al.** *Dev Dyn.,* October 1999, vol. 216 (2), 113-26
- **Zlotogorski et al.** Clinical and molecular diagnostic criteria of congenital atrichia with papular lesions. *J Invest Dermatol.,* 2002, vol. 118, 887-890
- **Ahmad et al.** Genomic organization of the humanhairless gene (HR) and identification of a mutation underlying congenital atrichia in the Arab Palestinian family. *Genomics,* 1999, vol. 56, 141-148
- **Sprecher et al.** Atrichia with popular lesions resulting from a nonsense mutation within the human hairless gene. *J. Invest. Dermatol,* 1999, vol. 113, 687-690
- **Ahmad et al.** A homozygous nonsense mutation in thezinc-finger domain of the human hairless gene underlines congenital atrichia. *J Invest Dermatol,* 1999, vol. 113, 281-283
- **Ahmad et al.** A miss sense mutation in the zinc-finger domain of the human hairless gene underlines congenital atrichia in a family of Irish travelers. *Am J Hum Genet,* 1998, vol. 63, 984-991
- **Aita et al.** A novel missense mutation (C622G) in the zinc-finger domain of the human hairless gene associated with congenital atrichia and popular lesions. *Exp Dermatol,* 2000, vol. 9, 157-162
- **Kruse et al.** Hairless mutations in two kindreds with autosomal recessive popular atrichia. *J Invest Dermatol,* 1999, vol. 113, 954-959
- **Zlotogorski et al.** Congenital atrichia in five Arab Palestinian families resulting from a deletion mutation in the human hairless gene. *Hum Genet.,* 1998, vol. 103, 400-404
- **Cichon et al.** Cloning, genomicorganization, alternative transcripts and mutational analysis of the gene responsible for autosomal recessive universal congenital alopecia. *Hum Mol Genet,* 1998, vol. 7, 1671-1679
- **Kruse et al.** Hairless mutations in two kindreds with autosomal recessive popular tarichia. *J Invest Dermatol,* 1999, vol. 113, 954-959
- **Klein et al.** A novel missense mutation affecting the human hairless thyroid receptor interaction domain 2 causes congenital atrichia. *J Invest Dermatol,* 2002, vol. 119, 920-922
- **Ahmad et al.** Alopecia universalis associated with mutation in the human hairless gene. *Science,* 1998, vol. 279, 720-724
- **Zlotogorski et al.** Evidence for pseudodominant inheritance of atrichia with papular lesions. *J Invest Dermatol,* 2002, vol. 118, 881-886
- **Sprecher et al.** Identification of a genetic defect in the hairless gene in atrichia with popular lesions: evidence for phenotypic heterogeneity among inheritedatrichia. *Am J Hum Genet,* 1999, vol. 64, 1323-1329
- **Nadler et al.** The Expression of adipogenic genes is decreased in obesity and diabetes mellitus. *Proc Natl Acad Sci (USA),* 2000, vol. 97, 11371-11376

- **Stulnig et al.** Novel roles of liver X receptors exposed by gene expression profiling in liver and adipose tissue. *Mol Pharmacol.,* 2002, vol. 62, 1299-1305
- **Stulnig et al.** Liver X receptors downregulate 11beta-hydroxysteroid dehydrogenase type 1 expression and activity. *Diabetes,* 2002, vol. 51, 2426-2433
- **Ross et al.** Microarray analyses during adipogenesis: understanding the effects of Wnt signaling on adipogenesis and the roles of liver X receptor alpha in adipocyte metabolism. *Mol Cell Biol,* 2002, vol. 22, 5989-5999
- **Matsuzaka et al.** Cloning and characterization of a mammalian fatty acyl-CoA elongase as a lipogenic enzyme regulated by SREBPs. *J Lipid Res,* 2002, vol. 43, 911-920
- **Zhang et al.** Regulation of lipoprotein lipase by the oxysterol receptors, LXRalpha and LXRbeta. *J Biol Chem,* 2001, vol. 276, 43018-43024
- **Yoshikawa et al.** Identification of liver X receptor-retinoid X receptor as an activator of the sterol regulatory element-binding protein 1c gene promoter. *Mol Cell Biol,* 2001, vol. 21, 2991-3000
- **Laffitte et al.** LXRs control lipid-inducible expression of the apolipoprotein E gene in macrophages and adipocytes. *Proc Natl Acad Sci U S A,* 2001, vol. 98, 507-512
- **M. Rechsteiner ; S. W. Rogers.** PEST Sequences and Regulation by Proteolysis. *TIBS,* 21 July 1996
- Vitamin D in Dermatology. Marcel Dekker, Inc, 2000